(19) Europäisches Patentamt
European Patent Office
Office européen des brevets

(11) EP 3 513 834 A1

(12) EUROPEAN PATENT APPLICATION

(43) Date of publication:
24.07.2019 Bulletin 2019/30

(51) Int Cl.:
A61N 1/05 (2006.01)          A61N 1/36 (2006.01)
A61N 1/372 (2006.01)         A61B 17/00 (2006.01)
A61B 17/34 (2006.01)

(21) Application number: 18185673.3

(22) Date of filing: 26.07.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Oticon Medical A/S
2765 Smørum (DK)

(72) Inventors:
• TOURREL, Guillaume
06220 Vallauris (FR)

• N'GUYEN, Yann
75013 Paris (FR)
• TORRES, Renato
75013 Paris (FR)
• FUNK, Rasmus
2300 København S. (DK)
• THOMSEN, Martin
2200 København N. (DK)

(74) Representative: William Demant
Oticon A/S
Kongebakken 9
2765 Smørum (DK)

(54) **SYSTEM FOR INSERTION OF A COCHLEAR ELECTRODE INTO THE COCHLEA**

(57) The invention relates to a system for insertion of a cochlear electrode into the cochlear of a hearing aid user, said system comprising a guiding tube configured to be inserted into the user, wherein a piston configured to displace the cochlear electrode along the guiding tube is slidably arranged in the guiding tube.

## Fig. 1A

**Description**

**FIELD**

The present disclosure relates to a system for insertion of a cochlear electrode into the cochlear of a hearing aid user.

**BACKGROUND**

Whereas conventional hearing aids capture, amplify and send sound through the normal auditory channel, a cochlearr implant, however, sends a signal directly to the auditory nerve. Accordingly, if an ear is too severely damaged, amplifying sound by using conventional hearing aids is not a suitable option. Unlike conventional hearing aids, cochlearr implants bypass the damaged areas of the ear. Cochlearr implants capture the sound, process it and electronically stimulate the auditory nerve.

Today, the routine in cochlearr implant is to manually insert the cochlearr electrode into the cochlear through the round window, or the cochleostomy. Surgeons normally use a micro forceps and a fine tip for carrying out this operation. Surgeons aim to position the electrode tip in front of the opening of the cochlear (round window or cochleostomy), and carefully push the electrode with the micro forceps. The surgeons use the fine tip in order to limit the lateral bending of the electrode and avoid the total kink of it. By this gestural, they laterally apply a force which help to insert the electrode.

During insertion of a cochlearr electrode, it is a major challenge to guide and push the electrode array into the right position. The trend in cochlearr implant is to design cochlearr electrode as small, smooth and flexible as possible, in order to be non-traumatic for the inner structure of the cochlear (basilar membrane, hair cells). Such electrodes are difficult to grab and push, without the risk of bending and folding the electrode array during insertion into the cochlear.

Therefore, there is a need to provide an improved insertion tool for inserting a cochlearr electrode into the cochlear.

**SUMMARY**

According to an aspect of the disclosure, the system is a system for insertion of a cochlear electrode into the cochlear of a hearing aid user, said system comprising a guiding tube configured to be inserted into the user, wherein a piston configured to displace the cochlear electrode along the guiding tube is slidably arranged in the guiding tube.

During insertion of a cochlearr electrode, it is a challenge to guide and push the electrode array into the right position. By using a piston slidably arranged in the guiding tube it is possible to guide and push the electrode array into the right position by means of the piston.

The guiding tube is configured to be inserted into the user and the piston is configured to displace the cochlear electrode along the guiding tube. The piston is shaped in such a manner that it can engage with and push the electrode array into the right position by means of the piston.

According to another aspect of the disclosure, a reinforced tube is arranged inside the guiding tube.

Hereby, the reinforced tube will guide the (flexible) piston so that the piston can push the cochlearr electrode into the system. Thus, the use of a reinforced tube allows for guidance of the cochlearr electrode.

The reinforced tube may include comprise any suitable material. In one embodiment, the guiding tube is a deformable guiding tube. It may be beneficial that a metallic reinforced tube is placed inside. In this embodiment, the system is a partial tube into another tube.

According to a further aspect of the disclosure, the reinforced tube is U-shaped. Hereby, it is possible to insert a wire prong into the reinforced tube. According to a further aspect of the disclosure, the reinforced tube is at least partly made of metal. The use of metal makes it possible to provide the required mechanical properties (large stiffness and strength) of the reinforced tube.

According to an even further aspect of the disclosure, the guiding tube is deformable. By having a deformable guiding tube, the guiding tube is capable of following the contour/geometry of the cochlear. By the term "deformable" is meant that the stiffness is sufficiently low to allow the guiding tube to be bent sufficiently.

According to yet aspect of the disclosure, the guiding tube is made in a plastic material. By making the guiding tube in a plastic material the guiding tube can be produced my injection moulding. Plastic injection moulding may include use of additive compositions such as reinforcement fibers or other structures used to provide any desired advantageous mechanical properties.

According to yet aspect of the disclosure, the guiding tube is deformable but rigid enough to guide the cochlear electrode without being deformed. Hereby, the guide tube can be used to guide the cochlear electrode into the desired position in a manner in which the guide tube will not collapse/be deformed.

According to another aspect of the disclosure, the guiding tube has a diameter in the range 0.4-0.9mm, such as 0.6-0.7mm, e.g. 0.65mm. These sizes have been found to be advantageous. Accordingly, they represent preferred embodiments of the guiding tube 20.

According to a further aspect of the disclosure, a slot is provided in the guiding tube. The slot makes it possible to push the cochlear electrode (as well as its silicone ring and the protective tubing) through the guiding tube, as it opens itself during insertion.

According to another aspect of the disclosure, an opening larger than the width of the slot is provided in proximal end of the slot. Hereby, depending on the position of the opening from the distal end of the guiding tube, the position of the cochlear electrode relatively to the distal end of the guiding tube can be controlled. It is possible to have a risen tip, or a recessed tip.

According to a further aspect of the disclosure, the piston comprises a prong structure comprising gripping members. The use of gripping members, 102' makes it possible to use the gripping members as abutting engagement structures when the piston is used for pushing a push ring.

In a preferred embodiment, the gripping members are configured to be arranged in:

a) an open configuration in which the distance between the gripping members is sufficiently large for the electrode to be loaded and in b) a closed configuration, in which the gripping structures are pressed together.

According to a further aspect of the disclosure, the piston comprises a U-shaped structure. Hereby, it is possible to apply the piston to push the cochlearr electrode into position.

According to another aspect of the disclosure, the cochlearr electrode is provided with a force sensor. Hereby, force measurements can be provided during the insertion and these measurements can be used to control the insertion forces. Accordingly, an improved insertion can be provided.

According to yet aspect of the disclosure, the system comprises an insertion tool comprising a handle and a visual insert depth indicator. Hereby, the visual insert depth indicator can provide information that makes it possible to carry out an improved insertion.

According to another aspect of the disclosure, the insertion tool comprises a motor and a push button arranged at the outside surface of the handle and being configured to activate the motor. Hereby, a user-friendly insertion tool can be provided. The surgeon can easily activate and hereby control the motor.

According to a further aspect of the disclosure, the system comprises a stabilizing unit. Hereby, the stabilizing unit can be used to mechanically support the insertion tool hereby providing a more stabile insertion.

According to yet aspect of the disclosure, the stabilizing unit is an arm member configured to be mechanically connected to the insertion tool. An arm member provides a simple and reliable way of supporting the insertion tool.

According to another aspect of the disclosure, the stabilizing unit is a static rotatable fixation member. The use of a static rotatable fixation member makes it possible to support the insertion tool in a manner in which the insertion tool can be fixed and rotated with respect to the stabilizing unit.

According to yet aspect of the disclosure, the system comprises a detection unit configured to measure the rate of change of insertion force during operation of the tool. Hereby, the detection unit can provide information that makes it possible to optimize the insertion. The surgeon can base the insertion on the rate of change of insertion force during on a continuous basis.

According to another aspect of the disclosure, the system comprises a control unit configured to make sure that insertion is continued until the real-time rate of change of force goes below or above a predefined quantity (threshold). Hereby, it is possible to provide an automatic insertion. By tracking the variation of insertion forces it is possible to provide a more safe and reactive automatized system which will sooner detect problems of insertion.

According to yet aspect of the disclosure, the predefined quantity (threshold) is a function of real time depth of insertion. Hereby, it is possible to provide a safer insertion.

According to another aspect of the disclosure, the force sensor is arranged in the casing of the insertion tool and comprises a one-axis sensor. Hereby, it is possible to apply the one-axis sensor to measure the overall generated forces in the insertion tool, in the direction of the movement of insertion. If the movement action is linear the sensor will measure a uniaxial force, whereas if the movement action is rotational the sensor will measure a torque.

According to yet aspect of the disclosure, the system is configured to measure the internal friction of the insertion tool. Hereby, the friction force which is assumed to be stable and reproducible according to the quality of the insertion tool manufacturing. When using the insertion tool device in real situation during inserting a cochlear electrode into a patient cochlear, the force measured by a force sensor will include the sum of the friction forces and the generated forces into the cochlear. By subtraction it is possible to provide the generated forces into the cochlear.

According to another aspect of the disclosure, the the system is configured to measure the internal friction of the insertion tool when the instrument is pushing the cochlearr electrode, but not in the cochlear.

According to yet aspect of the disclosure, the system is configured to apply the rate of change of deducted force to control insertion.

According to a further aspect of the disclosure, the system comprises a control unit configured to apply filtering to avoid false positives.

According to yet aspect of the disclosure, the system is configured to apply folding of the guiding tube and/or piston when the rate of change of deducted force exceeds a predefined value.

According to another aspect of the disclosure, the system comprises a force sensor provided with a rotational motor and wherein the system comprises a torque sensor.

**BRIEF DESCRIPTION OF DRAWINGS**

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

Fig. 1A    shows a schematic view of a prior art cochlearr implant device;
Fig. 1B    shows a perspective view of an insertion tool according to an embodiment of the disclosure;
Fig. 1C    shows a side view of the insertion tool shown in Fig. 1B;
Fig. 2    shows a side view of an insertion tool according to an embodiment of the disclosure;
Fig. 3A    shows an insertion tool according to an embodiment of the disclosure;
Fig. 3B    shows a close-up view of a guiding tube according to an embodiment of the disclosure;
Fig. 3C    shows an exploded view of some of the components of an insertion tool according to the disclosure;
Fig. 4    shows an exploded view of an insertion tool and guiding tube assemble according to an embodiment of the disclosure;
Fig. 5    shows an exploded view of a guiding tube assemble according to an embodiment of the disclosure;
Fig. 6A    shows a guiding tube assembly corresponding to the one shown in Fig. 4 and in Fig. 5 before insertion;
Fig. 6B    shows the guiding tube assembly shown in Fig. 6A, wherein a wire prong pushes the cochlearr electrode forward;
Fig. 6C    shows the guiding tube assembly shown in Fig. 6A, wherein an outer tube foil is retracted to disengage wire prong from the cochlearr electrode 4;
Fig. 6D    shows the guiding tube assembly shown in Fig. 6A, wherein the outer tube foil is transparent to show the placement of the components inside;
Fig. 7A    shows the proximal portion of a cochlearr electrode according to one embodiment of the disclosure;
Fig. 7B    shows the proximal portion of the cochlearr electrode shown in Fig. 7A;
Fig. 7C    shows the proximal portion of the cochlearr electrode shown in Fig. 7A;
Fig. 7D    shows the proximal portion of the cochlearr electrode shown in Fig. 7C;
Fig. 7E    shows the proximal portion of the cochlearr electrode shown in Fig. 7C;
Fig. 7F    shows the proximal portion of the cochlearr electrode shown in Fig. 7A;
Fig. 7G    shows the proximal portion of the cochlearr electrode shown in Fig. 7A;
Fig. 8A    shows a guiding tube in a first configuration and a second configuration;
Fig. 8B    shows a close-up view of the slider shown in Fig. 8A;
Fig. 8C    shows a cross-sectional view of the body portion, the slider and guiding tube show in Fig. 8B;
Fig. 9A    shows a guiding tube in a first configuration and a second configuration;
Fig. 9B    shows a close-up view of the sleigh and the protruding structure (slider) shown in Fig. 9A;
Fig. 9C    shows another close-up view of the sleigh and the protruding structure (slider) shown in Fig. 9A and in Fig. 9B;
Fig. 9D    shows a close-up view of the body portion arranged next to a flexible sleeve;
Fig. 10A    shows a graph depicting the insertion force as function of time during a manual insertion;
Fig. 10B    shows a graph depicting the insertion force as function of time during a guided insertion;
Fig. 10C    shows a graph depicting the insertion force as function of time T during an automatic insertion using a method according to one embodiment of the disclosure;
Fig. 10D    shows an insertion tool according to one embodiment of the invention;
Fig. 10E    shows a contact pad at the edge of the tube configured to keep in the right position the insertion tool;
Fig. 11A    shows a perspective view of an insertion tool according to an embodiment of the disclosure;
Fig. 11B    shows a perspective view of another insertion tool according to an embodiment of the disclosure;
Fig. 11C    shows a perspective view of another insertion tool according to an embodiment of the disclosure;
Fig. 12A    shows a schematic view of one embodiment of an automatized insertion tool according to the disclosure;
Fig. 12B    shows a graph depicting the force as insertion depth or time;
Fig. 12C    shows a graph depicting the force as insertion depth or time;

Fig. 13A    shows a graph depicting a sensor force, a friction force and deduced force versus insertion depth;
Fig. 13B    shows a graph depicting force versus insertion depth and
Fig. 13C    shows a close-up vie of a portion of the graph shown in Fig. 13B.

## DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlearr nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in a In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlearr Implant, or iv) arranging a unit of the hearing device as an entirely or partly implanted unit such as in Bone Anchored Hearing Aid or Cochlearr Implant. A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly running an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlearr Implant.

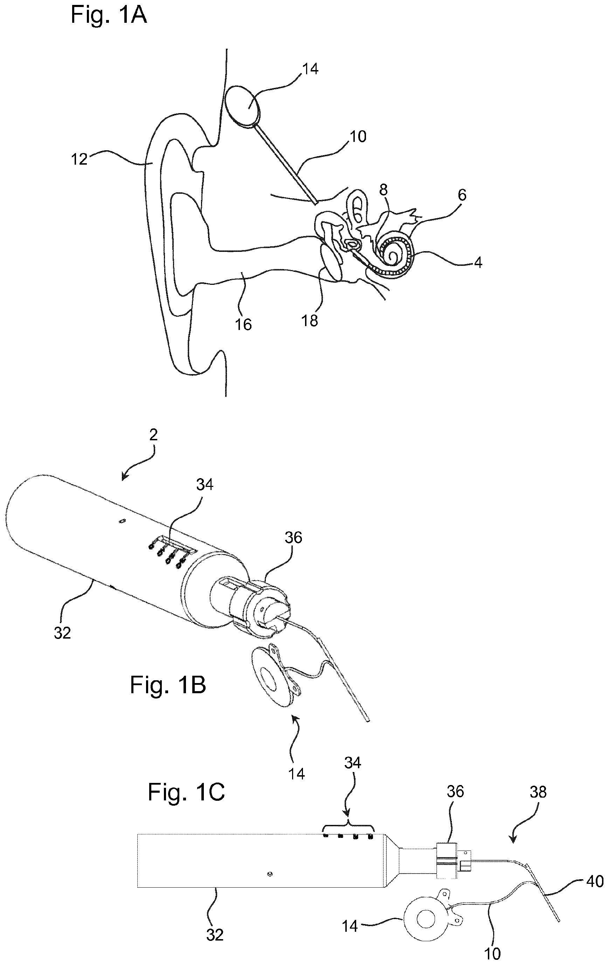

Now referring to Fig. 1A, which illustrates a schematic view of a prior art cochlearr implant (cochlearr stimulator) 14. A cochlearr lead 10 extends from the cochlearr implant 14 to the cochlearr 6, into which a cochlearr electrode (cochlearr electrode array) 4 is arranged. The proximal portion 8 of the cochlearr electrode 4 is indicated and its position is crucial for the function of the cochlearr implant 14. The ear 12, the ear canal 16 and the ear drum 18 of the person wearing the cochlearr implant 14 is shown.

Fig. 1B illustrates a perspective view of an insertion tool 2 according to an embodiment of the disclosure. Fig. 1C illustrates a side view of the insertion tool 2 shown in Fig. 1B. The insertion tool 2 comprises a casing 32 provided with an insertion depth indicator 34. The insertion depth indicator 34 can show the insertion depth on a visual scale during the insertion process. Accordingly, the user of the insertion tool 2 can receive information about the insertion depth on a continuous basis during the entire insertion process.

The insertion tool 2 comprises an interface 36 (for guiding tube replacement). A guiding tube 38 connected to an array 40 extends from the distal end of the interface 36. A cochlearr implant 14 is arranged in the free end of a cochlearr lead 10 extending from the distal end of the array 40. Fig. 1C illustrates a first step of the insertion of a cochlearr electrode (electrode array) 4 into the cochlearr 6. In this first step, the proximal portion 8 of the cochlearr electrode 4 is arranged in a first position I.

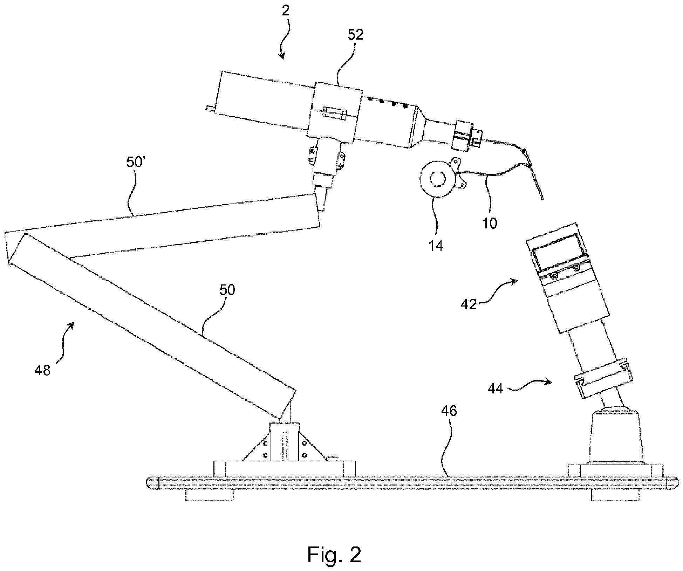

Fig. 2 illustrates a side view of an insertion tool 2 according to an embodiment of the disclosure. The insertion tool 2 corresponds to the one shown in Fig. 1B and Fig. 1C. The insertion tool 2 is arranged in a holding assembly 52. The holding assembly 52 comprises an attachment portion that is rotatably attached to a positioning arm 48 comprising a first segment 50 that is rotatably attached to a base structure in its proximal end and to a second segment 50' in its distal end. The base structure rests on a base plate 46 provided with feet for engagement with a surrounding structure (e.g. a table). A cochlear mock-up 42 is arranged at the distal end of an adjustment member 44 that is attached to the plate 46.

When the insertion tool 2 has to be used in situ, the cochlear mock-up 42 would be removed. Furthermore, the positioning arm 48 would preferably be positioned in a manner in which the there is access to the head of the person into whom the cochlearr electrode has to be inserted.

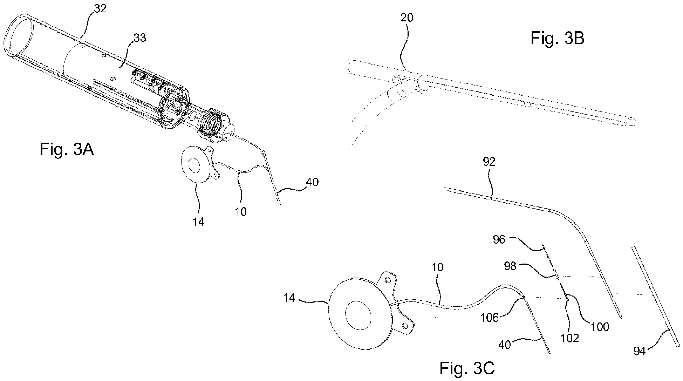

Fig. 3A illustrates an insertion tool 2 according to an embodiment of the disclosure. The casing 32 is made transparent for allowing the internal components to be visible. It can be seen that an inner portion 33 is slidably arranged within the casing 32: Accordingly, the inner portion can be displaced along the length of the cylindrical casing 32. This may be accomplished by using an actuator (not shown), preferably an electrical actuator. An applicable electrical actuator may be an electrical motor.

The insertion tool 2 comprises an interface for guiding tube replacement. A cochlearr implant 14 is arranged in the free end of a cochlearr lead 10 extending from the distal end of the cochlearr lead 10. Fig. 3B illustrates a close-up view of a guiding tube 20 according to an embodiment of the disclosure without an outer tube foil.

Fig. 3C illustrates an exploded view of some of the components of an insertion tool according to the disclosure. A cochlearr implant (cochlearr stimulator) 14 is arranged in the distal end (free end) of a cochlearr lead 10 extending from the distal end of an array 40. The insertion tool comprises a flexible piston member 96 configured to be arranged in a wire crimp 98. The insertion tool moreover comprises a wire prong 100 configured to be arranged in the wire crimp 98.

In one embodiment according to the disclosure, the wire crimp 98 is formed as a cylindrical tubular structure comprising a first end for receiving said piston member 96 and a second end configured for receiving said wire prong 100.

The wire prong 100 comprises an attachment structure/gripping member 102 provided in the distal end of the wire prong 100. Said attachment structure/gripping member 102 is configured to engage with a corresponding flange structure 106 of the cochlearr lead 10.

The insertion tool moreover comprises an outer tube 92 configured to receive the wire crimp 98 (and the structures 96, 100 attached thereto). The insertion tool furthermore comprises an outer tube foil 94 configured to cover the outer tube 92.

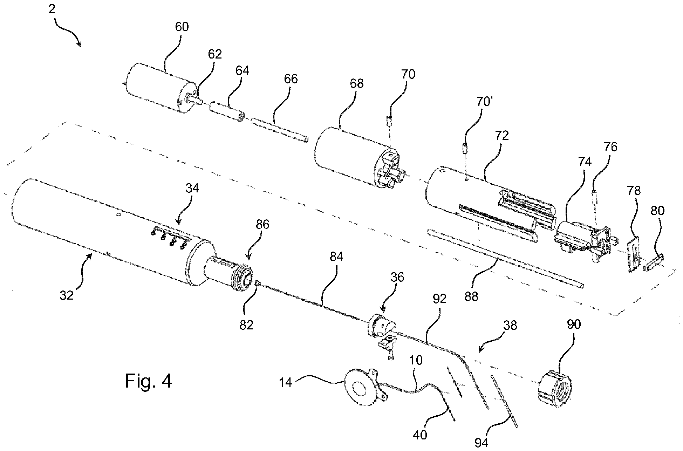

Fig. 4 illustrates an exploded view of an insertion tool 2 and guiding tube assemble 38 according to an embodiment of the disclosure. The insertion tool 2 corresponds to the one shown in Fig. 1B, Fig. 1C, Fig. 2 and Fig. 3A. The insertion tool comprises a casing (outer chassis) 32 provided with an insertion depth indicator 34. A sensor interface 82 configured to be arranged in the structure provided in the structure having a threaded portion 86 and protruding from the casing 32.

A rigid piston member 84 is arranged in front of said treaded portion 86. A guiding tube interface 36 is arranged and configured to be attached to or abut said a threaded portion 86.

A guiding tube assemble 38 comprising an outer tube 92 is arranged in front of the guiding tube interface 36. The guiding tube assemble 38 corresponds to the one shown in Fig. 3C. A screw cap 90 is arranged in front of the guiding tube assemble 38. The screw cap 90 is configured to be attached to the treaded portion 86 by screwing a corresponding engaging inner treaded portion thereto.

A cochlearr implant 14 (cochlearr stimulator) arranged in the distal end of a cochlearr lead 10 extending from the distal end of an array 40 is provided below the guiding tube interface 36.

The inside components of the casing 32 is shown above the casing 32. The inside components include an electric motor 60 provided with a centrally arranged shaft comprising an outer portion protruding from the end portion of the motor 60.

A cylindrical tubular joint 64 configured to be attached to the shaft 62 is arranged next to the shaft 62. A spindle 66 configured to be mechanically attached to the shaft 62 via the joint 64 is shown. A motor mount 68 configured to receive the electric motor 60. The motor mount 68 is provided with an opening through which the spindle 66 can extend when the electric motor 60 is received by the motor mount 68.

A motor mount guide 72 is arranged next to the motor mount 68. The motor mount guide 72 has a cylindrical portion provided with an opening for receiving said motor mount 68. The motor mount 68 is configured to be attached to the motor mount guide 72 by means of screws 70, 70'.

A piston 74 is arranged in front of the motor mount guide 72. The piston 74 comprises engagement structures corresponding to receiving portions of the motor mount guide 72. Accordingly, the motor mount guide 72 is configured to receive the piston 74. When the piston 74 is arranged/received in the receiving portions of the motor mount guide 72, the piston 74 can be displaced along the length of the motor mount guide 72. A force sensor 78 is configured to be attached to proximal portion of the piston 74. A piston bridge 80 is configured to be arranged in a manner in which the force sensor 78 can be sandwiched between the proximal portion of the piston 74 and the piston bridge 80.

A cable guide 88 configured to be inserted into the motor mount guide 72. An insertion depth indication pin 76 is arranged above the piston 74 for being inserted into a receiving hole provided in the piston 74.

Rotation of the motor 60 will rotate the spindle 66. This will cause translation of the piston 74 along the length of the motor mount guide 72 since the spindle 66 is rotatably attached to the piston 74 by means of a receiving portion comprising an inner treaded portion corresponding to the outer threads of the spindle 66 (not shown).

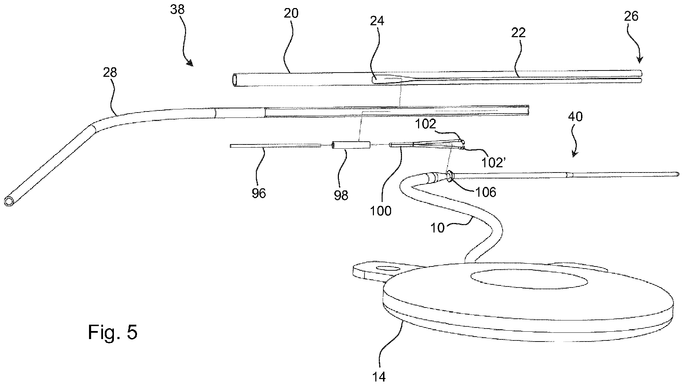

Fig. 5 illustrates an exploded view of a guiding tube assemble 38 according to an embodiment of the disclosure. The guiding tube assemble 38 comprises a guiding tube 20 provided with an opening 24 and a slot 22 through which a reinforcement tube 28 can be introduced. The guiding tube 20 comprises a distal end 26.

A piston 96 is arranged to be inserted into a wire prong crimp 98, wherein said wire prong crimp 98 is configured to receive the proximal end of a wire prong 100 provided with two gripping members 102, 102' in its distal end. The wire prong crimp 98 is configured to be arranged inside the reinforcement tube 28.

A cochlearr implant 14 is arranged in the proximal end of a cochlearr lead 10 extending from said cochlearr implant 14. The

The attachment structures/gripping members 102, 102' provided in the distal end of the wire prong 100 are configured to engage with the corresponding flange structure 106 of the cochlearr lead 10. Accordingly, movement of the piston 96 will cause displacement of the wire prong 100 and thus the flange structure 106. Therefore, the array 40 can be displaced/moved into the desired position within the cochlear.

The insertion tool moreover comprises an outer tube 92 configured to receive the wire crimp 98 (and the structures 96, 100 attached thereto). The insertion tool furthermore comprises an outer tube foil 94 configured to cover the outer tube 92.

In case of full insertion, when finishing the insertion, the wire prong 100 protrudes completely out of the guiding tube 20 and thus it takes its "open" position and releases the electrode array 40. The insertion tool can be removed.

In case of partial insertion, the electrode array 40 remains blocked into the guiding tool. For releasing it, the guiding tube 20 can slide back independently to the rest of the components and system. Consequently, the wire prong 100 is releasing and the electrode array 40 too.

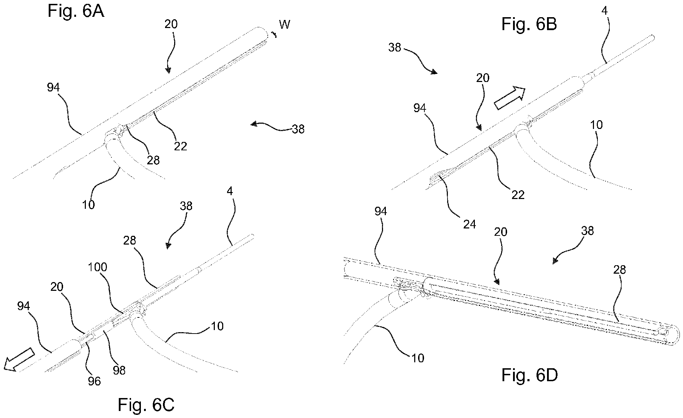

Fig. 6A illustrates a guiding tube assembly 38 corresponding to the one shown in Fig. 4 and in Fig. 5 before insertion. Fig. 6B illustrates the guiding tube assembly 38 shown in Fig. 6A, wherein a wire prong pushes the cochlearr electrode 4 forward. Fig. 6C illustrates the guiding tube assembly 38 shown in Fig. 6A, wherein an outer tube foil is retracted to disengage wire prong 100 from the cochlearr electrode 4. Fig. 6D illustrates the guiding tube assembly 38 shown in Fig. 6A, wherein the outer tube foil 94 is transparent to show the placement of the components inside.

In Fig. 6A, it can be seen that the opening 28 is larger than the width W provided in proximal end of the slot 22. The cochlearr lead 10 extends basically perpendicular to the length of the guiding tube 20 and the outer tube foil 94 surrounding it. An opening/ loading zone 24 (shown in Fig. 6B) is provided in the outer tube foil 94 and the cochlearr electrode is placed inside the guiding tube 20. Accordingly, the cochlearr electrode is ready to be inserted into the cochlearr of a hearing-impaired person. The guiding tube 20 is provided with a fine slot 22 extending along the length of the guiding tube 20, between the opening/loading zone 24 and the proximal end of the guiding tube 20. Initially, the guiding tube 20 with its slot 22 remains closed. Due to mechanical properties (flexibility) of the soft material used for the guiding tube 20 and the slot 22, it is possible to push the cochlearr electrode, its push ring (preferably a silicone ring) and the protective tubing through the guiding tube 20, as it opens itself during insertion. Tests have revealed that the generated friction forces are sufficiently small to control the insertion.

In Fig. 6B the wire prong has pushed the cochlearr electrode forward along the direction indicated by an arrow.

In Fig. 6C the guiding tube 20 has been slid back (along the direction of the arrow). Hereby, it is possible to partially release the cochlearr electrode 4 hereby allowing the cochlearr electrode 4 to be inserted by using the pushing function illustrated in Fig. 6C. Accordingly, the wire prong 100, the wire prong crimp 98 and piston member 96 are visible.

To limit the problem of a too large diameter for the rigid metallic guiding tube in the prior art, one preferred embodiment of the disclosure uses a plastic and deformable guiding tube 20, rigid enough to sufficiently guide the cochlearr electrode 4, with a diameter only 0.2mm more than the active cochlearr electrode 4 of 0.5mm. In Fig. 6D it can be seen that the reinforced tube 28 is U-shaped. The reinforced tube 28 preferably comprises a metallic material. In one embodiment, the reinforced tube 28 is U-shaped and made of metal.

The system comprises a partial (U-shaped) tube arranged inside another tube. The function of the reinforced tube 28 is also to guide the flexible piston member 96 that is configured and arranged to push the cochlearr electrode 4 into the system. This configuration allows perfect guidance of the cochlearr electrode 4, especially at the tip, but also all along the cochlearr electrode 4. Accordingly, it limits the wave effect into the tube.

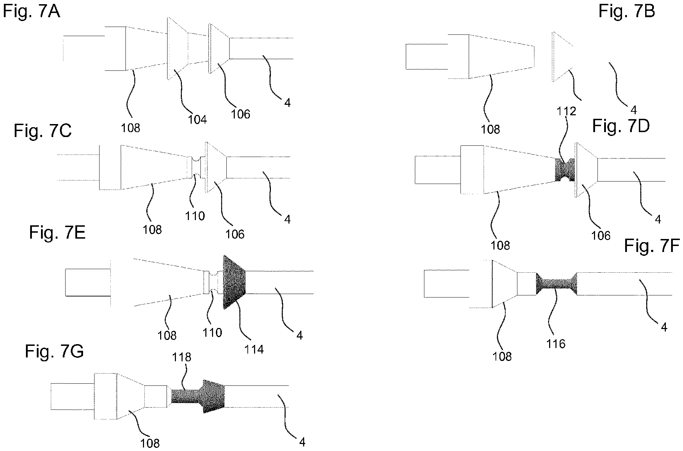

Fig. 7A illustrates the distal portion of a cochlearr electrode 4 according to one embodiment of the disclosure. A conical body portion 108 is attached to the cochlearr electrode 4. A rear push ring 104 is provided on the body portion 108. A front push ring 106 is provided at the distal end of the body portion 108.

Fig. 7B illustrates the distal portion of the cochlearr electrode 4 shown in Fig. 7A. The rear push ring, however, has been removed.

Fig. 7C illustrates the distal portion of the cochlearr electrode 4 shown in Fig. 7A. The rear push ring has been removed and a recess 110 for a wire prong has been added.

Fig. 7D illustrates the proximal portion of the cochlearr electrode 4 shown in Fig. 7C. An over moulded insert 112 has been added to the recess 110.

Fig. 7E illustrates the proximal portion of the cochlearr electrode 4 shown in Fig. 7C. The front push ring has been changed to an over moulded (preferably injection molded) part 114.

Fig. 7F illustrates the proximal portion of the cochlearr electrode 4 shown in Fig. 7A, wherein the rear push ring and the front push ring have been removed and a recess 116 for wire prong provided as an over moulded (preferably injection moulded) part has been added.

Fig. 7G illustrates the proximal portion of the cochlearr electrode 4 shown in Fig. 7A, wherein the rear push ring and the front push ring have been removed and a recess and smaller push ring provided as an over moulded (preferably injection moulded) part/insert 118 has been added.

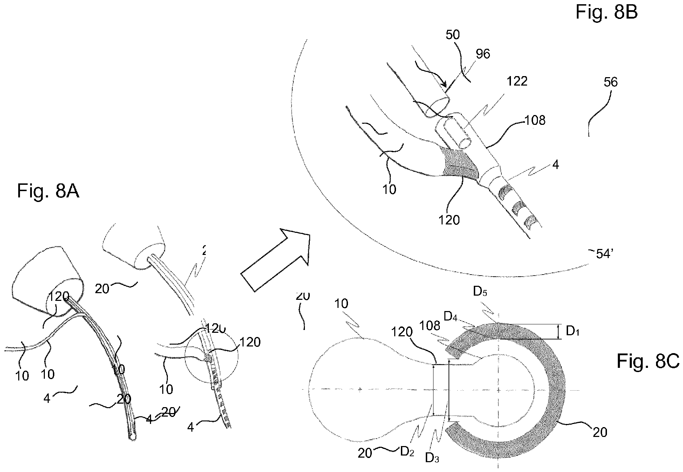

Fig. 8A illustrates a guiding tube 20 in a first configuration, in which the cochlearr lead 4 protrudes from a portion near the proximal end of the guiding tube 20 and in which the guiding tube 20 is almost entirely closed; and a second configuration, in which the cochlearr lead 10 protrudes from a portion near the distal end of the guiding tube 20 and in which the guiding tube 20 has been opened much more than in the first configuration by using a slider 120 protruding from a body portion provided at the end of the cochlearr electrode 4.

Fig. 8B illustrates a close-up view of the slider 120 shown in Fig. 8A. It can be seen that the slider 120 protrudes from a body portion 108 provided with a cylindrical insert 122. The insert 122 is preferably made of metal. The insert 122 is centrally arranged with respect to the longitudinal axis of the body portion 108 and is arranged at the free end thereof. The slider 120 is configured to thread its way through the slot in the guiding tube 20 and hereby widen up the slot in the guiding tube 20. It may be an advantage that the slider 120 is sharp or has a limited edge thickness. A piston member 96 is arranged next to the body portion 108.

Fig. 8C illustrates a cross-sectional view of the body portion 108, the slider 120 and guiding tube 20 show in Fig. 8B. It can be seen that the slide 120 has a dimension (thickness) $D_2$ that is smaller than the width $D_3$ of the slot in the guiding tube 20. The thickness $D_1$ of the guiding tube 20, the inner diameter $D_4$ and the outer diameter $D_5$ of the guiding tube 20 is indicated.

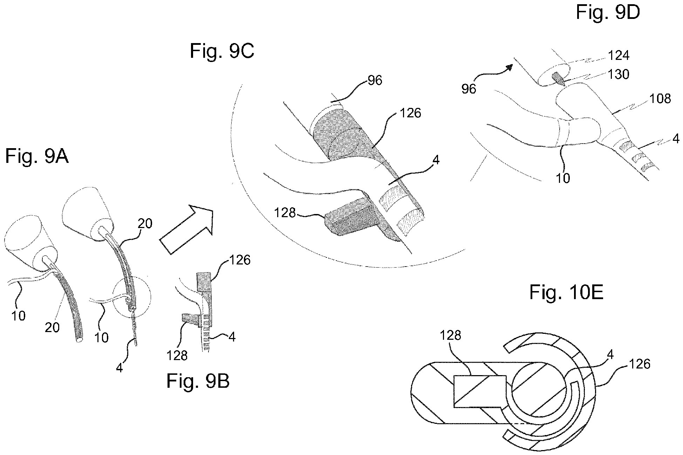

Fig. 9A illustrates a guiding tube 20 in a first configuration, in which the cochlearr lead 4 protrudes from a portion near the proximal end of the guiding tube 20 and in which the guiding tube 20 is almost entirely closed; and a second configuration, in which the cochlearr lead 10 protrudes from a portion near the distal end of the guiding tube 20 and in which the guiding tube 20 has been opened much more than in the first configuration by using a protruding structure (a slider) 128 protruding from a sleigh for holding the cochlearr electrode 4.

Fig. 9B illustrates a close-up view of the sleigh 126 and the protruding structure (slider) 128 shown in Fig. 9A. It can be seen that the sleigh 126 is configured to hold the cochlearr electrode 4 and that the protruding structure 128 extends basically perpendicular to the length of the sleigh 126.

Fig. 9C illustrates another close-up view of the sleigh 126 and the protruding structure (slider) 128 shown in Fig. 9A and in Fig. 9B. It can be seen that a piston member 96 is arranged next to the free end of the sleigh 126. Accordingly, displacement of the piston member 96 towards the sleigh 126 Will move the sleigh 126 ahead.

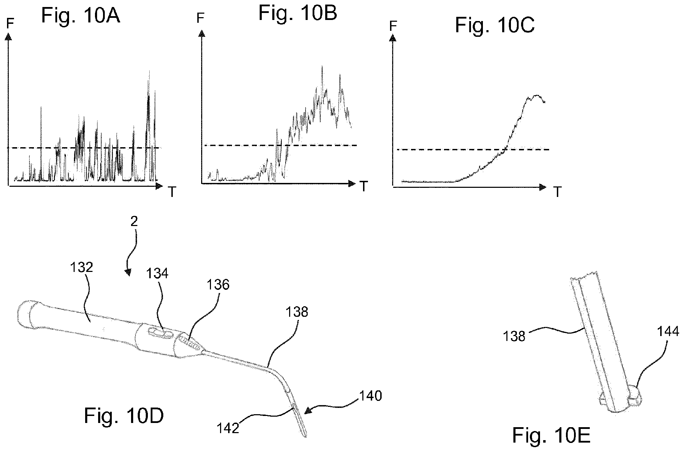

Fig. 9D illustrates a close-up view of the body portion 108 arranged next to a flexible sleeve 124. A retractable pointed pin 130 is centrally arranged in the end portion of the sleeve 124 of the piston member 96. The pointed pin 130 extends axially in extension of the sleeve 124 and is configured to push the body portion 108 and the cochlearr electrode 4 forward into the desired position in the cochlearr. The pointed pin 130 is placed at the tip of the piston member 96 and comes in contact at the base of the body portion 108 arranged next to the cochlearr electrode 4. The pointed pin 130 id arranged and configured to avoid occurrence of a possible misalignment during the pushing phase. In case of partial insertion, the pointed pin 130 also allows pull back movement of the cochlearr electrode 4. The body portion 108 is arrange din the proximal end of the cochlearr electrode 4. A cochlearr lead 10 (lateral wire output) protrudes from the body portion 108. The proximal portion of cochlearr lead 10 extends basically perpendicular to the body portion 108. Fig. 10E illustrates a cross-sectional view of the cochlearr electrode 4 surrounded by the sleigh 126 shown in Fig. 9C. The protruding structure (slider) 128 is also shown.

Fig. 10A illustrates a graph depicting the insertion force F as function of time T during a manual insertion. It can be seen that the insertion force F fluctuates very much.

Fig. 10B illustrates a graph depicting the insertion force F as function of time T during a guided insertion. It can be seen that the insertion force F fluctuates less than the insertion force F during the manual insertion shown in Fig. 9A.

Fig. 10C illustrates a graph depicting the insertion force F as function of time T during an automatic insertion using a method according to one embodiment of the disclosure. It can be seen that the insertion force F fluctuates less than the insertion force F during both the manual insertion shown in Fig. 9A and the guided insertion shown in Fig. 9B.

Fig. 10D illustrates an insertion tool 2 according to one embodiment of the invention. The insertion tool 2 is formed as a hand piece comprising a handle 132 having a distal end in which a push button 134 and a visual indicator (configured to indicate the insertion depth) is provided. The hand piece is basically shaped as a pen.

A tube 138 extends in extension from the proximal portion of the handle 132- The tube 138 comprises a bent portion. A sliding foil 140 is provided at the proximal portion of the tube 138. A slot 142 is extends along the length of the last part of the proximal portion of the tube 138.

The primary battery, the motor and the sensor are integrated into the handle 132. On the distal part of the handle 132 the push button 134 id arranged to control the advance front and back of a cochlearr electrode into its guiding tube. Preferably the visual indicator 136 comprises a liquid crystal display (LCD) or a light-emitting diode (LED) being integrated on the distal edge of the handle 132. In a preferred embodiment, the complete system is disposable and can be sterilized. In a preferred embodiment, the insertion tool 2 is arranged in a packing and being sterilized and thus ready to be used in the surgical room. Accordingly, the surgeon or his assistant will initially unpack the insertion tool 2. Hereafter, the surgeon or his assistant can load the cochlearr electrode 4 (electrode array) into the distal part of the tube 138, into the slot 142 of the sliding foil 140. After this, the surgeon can place the edge of the tube 138 in front of the cochleostomy, and maintains manually this position during the insertion phase.

As shown in Fig. 10E, the contact pad 144 at the edge of the tube 138 (guiding tube) helps to keep in the right position the insertion tool 2, and cancels the tremors normally induced by the manual handling of the surgical tool. The contact pad 144 is configured to be brought into contact with the external bone of the cochlear during insertion of the cochlearr electrode 4 into the cochlearr.

When the insertion is finished and when the cochlearr electrode 4 (electrode array) has been entirely or partially inserted into the cochlear, the surgeon can slide back the sliding foil 140, and so release the cochlearr electrode 4 connected to the stimulator. The surgeon can throw away the disposable insertion tool 2 and finish the surgery as normally.

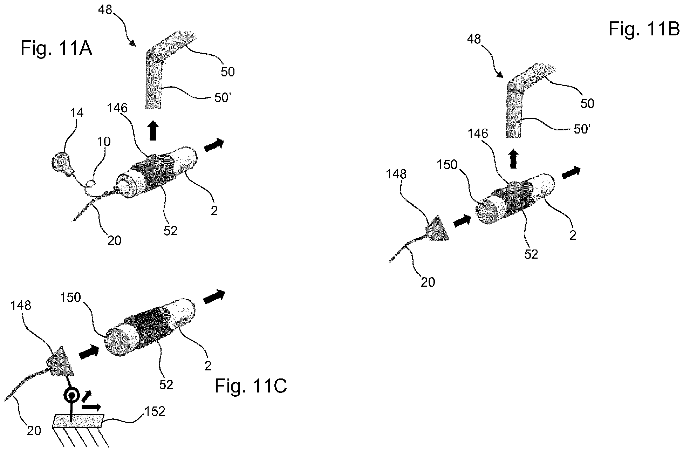

Fig. 11A illustrates a perspective view of an insertion tool 2 according to an embodiment of the disclosure arranged to be suspended in a positioning arm 48 comprising a first segment 50 and a second segment 50' rotatably attached thereto.

The insertion tool 2 is arranged in a holding assembly 52 provided with a fixation structure 146 arranged and configured to be attached to the distal end og the second segment 50'. The insertion tool 2 comprises a guiding tube 20 extending in extension from the proximal portion of the insertion tool 2. A cochlearr implant 14 provided with a cochlearr lead 10 is attached to the guiding tube 20 by means of the cochlearr lead 10.

Fig. 11B illustrates a perspective view of another insertion tool 2 according to an embodiment of the disclosure arranged to be suspended in a positioning arm 48 comprising a first segment 50 and a second segment 50' rotatably attached thereto. The insertion tool 2 comprises a disposable guiding tube structure 148 that is configured to be detachable from the insertion tool 2 that constitutes a hand piece comprising a motor. The disposable guiding tube structure 148 is configured to be attached to an interconnection interface 150 provided on a closed receptacle.

Fig. 11C illustrates a perspective view of another insertion tool 2 according to an embodiment of the disclosure. The insertion tool 2 corresponds to the one shown in Fig. 11B. However, the positioning arm 48 is replaced by a fixation member 152 arranged under the insertion tool 2. The fixation function is included in the interconnection interface 150, and not on the hand piece. As schematised, the fixation member 152 enables movements with many degrees of freedom. In one preferred embodiment, the fixation member 152 enables movements is configured to enable at least one rotation and translations in three orthogonal directions. This configuration allows to easily position in the right and accurate place the distal edge of the guiding tube 20 in front of the cochleostomy, and allows a later connection of the hand piece.

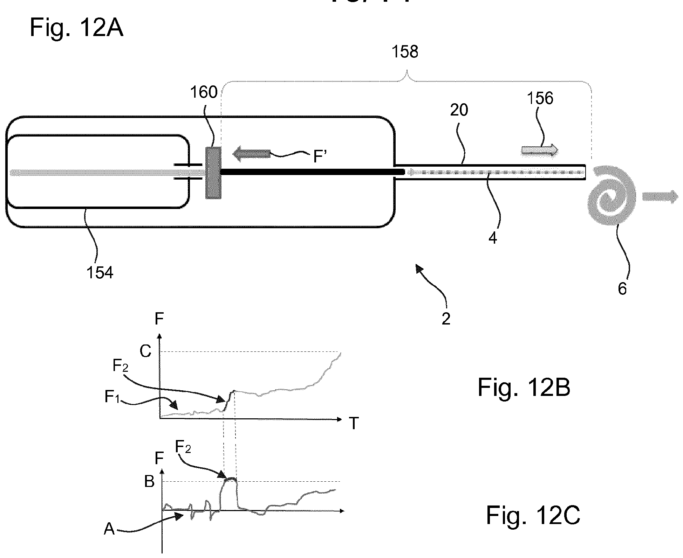

Fig. 12A illustrates a schematic view of one embodiment of an automatized insertion tool 2 according to the disclosure. The insertion force measurement principle is illustrated. An overview of the system 158 is shown in Fig. 8B. The functioning principle of the insertion tool is to indirectly measure the forces generated into the cochlear 6. As it's evident that no sensor can measure the forces directly into the patient cochlear 6, the insertion tool comprises at least a one axis sensor 160 measuring the overall generated forces in the insertion tool 2, in the direction of the movement of insertion 156.

In one embodiment, the movement action is linear as indicated in Fig. 12A, and the sensor 156 is configured to measure a uniaxial force F'.

In another embodiment, the movement action is rotational, and the sensor is configured to measure a torque.

In Fig. 12A it can be seen that the insertion tool 2 comprises an electric motor (preferably a linear motor) 154 and a sensor 160 that is integrated into the housing of the system 160. The motor 154 is configured to create a motion that pushes the cochlearr electrode 4 inside the guiding tube 20 forward in the indicated direction of movement 156 into the cochlearr 6. The cochlearr electrode 4 is a flexible (non-rigid) electrode array arranged to be inserted into the cochlear 6. The measured uniaxial force measured by the sensor 160 includes generated forces into the cochlear 6 as well as the induced friction forces into the system 158.

As shown in Fig. 12B, it is possible to visualize the forces on the graph as a function of insertion depth into the cochlear 6. The induced friction forces into the system is plotted and is a proper characteristic of the device and the electrode array pushed inside it. The induced friction forces into the system is therefore measure during an insertion "in the air", or without any cochlear 6. The friction forces are assumed to be stable and reproducible according to the quality of the device manufacturing. When using the insertion tool device in real situation, for inserting a cochlearr electrode 4 into a patient cochlear 6, the force measured by the sensor 160 will measured the cumulated forces; the friction forces and the generated forces into the cochlear 6. By subtraction of both forces, we can deduct the generated forces alone into the cochlear 6.

Calibration of the insertion tool 2 can be carried out in facility during the design and after as a manufacturing monitoring to avoid any parameter drift, the characteristic friction forces can be save in the system together with the name of the configuration in order to be later called by the user. Each configuration of an insertion tool and version of a cochlearr electrode 4 must initially calibrated in facility in order to determine the behaviour. This means that after calibrating and knowing the insertion device, its behaviour is independent to the cochlear 6 wherein the cochlearr electrode 4 will be inserted. The calibration means in this case can be done by carrying out a plurality of "insertions" using the insertion tool 2 with a cochlearr electrode 4 "in the air". These several trials must then be compiled. Hereafter, it is possible to calculate a mean curve as well as the envelope curve.

By monitoring, during the cochlearr electrode 4 insertion, not only the maximum force allowable, but additionally tracking the variation of insertion forces instead of only the absolute force value, it is possible to provide a more safe and reactive automatized system which will sooner detect problems of insertion which can't be anticipated even with a library of use cases.

In one first step, continuous measurement of the uniaxial force with the sensor is carried out. Hereafter, it is possible to compute and deduct the induced forces into the cochlear. Knowing the insertion speed as it's a controlled parameter which can be modified by the surgeon, it is possible to calculate the slope value at any time.

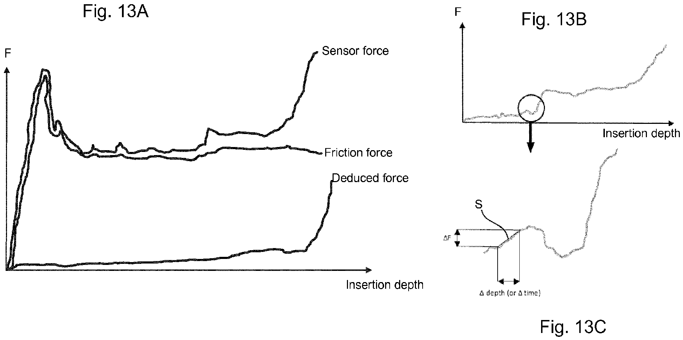

This calculation corresponds to a first order derivative of the insertion force. As it's a function of insertion speed, it can be integrated to the time or the insertion length. As shown in Fig. 13C, the slope value S is continuously computed: by using the following equation:

$$(a) \qquad \text{Slope } S = \Delta F / \Delta \text{ insertion depth.}$$

The sensibility of the slope calculation can be adapted by chosen a different pitch $\Delta$ depth.

Fig. 12B illustrates a graph depicting the force F as insertion depth or time T.

Fig. 12C illustrates a graph depicting the force F as insertion depth or time T. Force F is a first order derivative function of force.

The dynamic slope value A may be displayed in superposition of the absolute insertion force $F_1$, or on a secondary screen. A threshold limit B can be set in order to detect a too high slope value C coming from a too fast force rising $F_2$.

Filtering can be applied to avoid untimely detection of small drift and inappropriate insertion stop.

Fig. 13A illustrates a graph depicting a sensor force, a friction force and deduced force versus insertion depth. Thus, it is possible to visualize the forces on the graph as a function of insertion depth into the cochlear. The induced friction forces into the system is plotted and is a proper characteristic of the insertion tool and the cochlearr electrode pushed inside it. Accordingly, the induced friction forces into the system is measured during an insertion "in the air", or without any cochlear. These friction forces are assumed to be stable and reproducible according to the quality of the device manufacturing. When using the insertion tool device in real situation, for inserting a cochlearr electrode into a patient cochlear, the force measured by the sensor will measured the cumulated forces; the friction forces and the generated forces into the cochlear. By subtraction of both forces, we can deduct the generated forces alone into the cochlear.

As shown in Fig. 13B and Fig. 13C, the slope value S is continuously computed by using the previously mentioned equation

$$\text{(a)} \qquad \text{Slope } S = \Delta F / \Delta \text{ insertion depth.}$$

A cochlearr implant typically includes i) an external part for picking up and processing sound from the environment, and for determining sequences of pulses for stimulation of the electrodes in dependence on the current input sound, ii) a (typically wireless, e.g. inductive) communication link for simultaneously transmitting information about the stimulation sequences and for transferring energy to iii) an implanted part allowing the stimulation to be generated and applied to a number of electrodes, which are implantable in different locations of the cochlear allowing a stimulation of different frequencies of the audible range. Such systems are e.g. described in US 4,207,441 and in US 4,532,930.

In an aspect, the hearing device comprises multi-electrode array e.g. in the form of a carrier comprising a multitude of electrodes adapted for being located in the cochlear in proximity of an auditory nerve of the user. The carrier is preferably made of a flexible material to allow proper positioning of the electrodes in the cochlear such that the electrodes may be inserted in cochlear of a recipient. Preferably, the individual electrodes are spatially distributed along the length of the carrier to provide a corresponding spatial distribution along the cochlearr nerve in cochlear when the carrier is inserted in cochlear.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more. Accordingly, the scope should be judged in terms of the claims that follow.

**List of reference numerals**

| | |
|---|---|
| 2 | Insertion tool |
| 4 | Cochlearr electrode |

| | |
|---|---|
| 6 | Cochlear |
| 8 | Proximal portion |
| 10 | Cochlearr lead |
| 12 | Outer ear |
| 14 | Cochlearr implant |
| 16 | Ear canal |
| 18 | Eardrum |
| 20 | Guiding tube |
| 22 | Slot |
| 24 | Opening |
| 26 | Distal end |
| 28 | Reinforcement tube |
| 30 | Piston |
| 32 | Casing |
| 33 | Inner portion |
| 34 | Insertion depth indicator |
| 36 | Interface (for guiding tube replacement) |
| 38 | Guiding tube assembly |
| 40 | Array |
| 42 | Cochlear mock-up |
| 44 | Adjustment member |
| 46 | Base plate |
| 48 | Positioning arm |
| 50, 50' | Segment |
| 52 | Holding assembly |
| 54, 54' | Cable |
| 56 | Monitor |
| 58 | Actuation interface |
| 60 | Motor |
| 62 | Shaft |
| 64 | Joint |
| 66 | Spindle |
| 68 | Motor mount |
| 70, 70' | Screw |
| 72 | Motor mount guide |
| 74 | Piston |
| 76 | Pin |
| 78 | Force sensor |
| 80 | Piston bridge |
| 82 | Sensor interface |
| 84 | Rigid piston member |
| 86 | Treaded portion |
| 88 | Cable guide |
| 90 | Screw cap |
| 92 | Outer tube |
| 94 | Outer tube foil |
| 96 | Piston member |
| 98 | Wire prong crimp |
| 100 | Wire prong |
| 102, 102' | Gripping member/attachment structure |
| 104 | Push ring |
| 106 | Flange structure/push ring |
| 108 | Body portion |
| 110 | Recess |
| 112, 114, 116, 118 | Over moulded part |
| 120 | Slider |
| 122 | Insert (preferably made of metal) |
| 124 | Flexible sleeve |

| 126 | Sleigh for holding assembly |
|---|---|
| 128 | Protruding structure |
| 130 | Pin member |
| 132 | Handle |
| 134 | Push button |
| 136 | Visual indicator |
| 138 | Tube |
| 140 | Sliding foil |
| 142 | Slot |
| 144 | Contact pad |
| 146 | Fixation structure |
| 148 | Disposable guiding tube structure |
| 150 | Interconnection interface |
| 152 | Fixation member |
| I, II, III, IV | Position |
| W | Width |
| $D_1, D_2, D_3, D_4, D_5$ | Distance |
| F, F' | Force |
| T | Time |
| A | Dynamic slope value |
| B | Threshold limit |
| C | High slope value |
| S | Slope |
| $F_1$ | Absolute insertion force |
| $F_2$ | Fast force rising |

**Claims**

1. A system for insertion of a cochlear electrode (4) into the cochlear (6) of a hearing aid user, said system comprising a guiding tube (20) configured to be inserted into the user, wherein a piston (30) configured to displace the cochlear electrode (4) along the guiding tube (20) is slidably arranged in the guiding tube (20).

2. A system according to claim 1, wherein a reinforced tube (28) is arranged inside the guiding tube (20).

3. A system according to claim 2, wherein the reinforced tube (28) is U-shaped.

4. A system according to one of the preceding claims, wherein the guiding tube (20) is deformable.

5. A system according to one of the preceding claims, wherein a slot (22) is provided in the guiding tube (20).

6. A system according to claim 5, wherein an opening (28) larger than the width (W) of the slot (22) is provided in proximal end of the slot (22).

7. A system according to one of the preceding claims, wherein the piston (30) comprises a prong structure (100) comprising gripping members (102, 102').

8. A system according to one of the preceding claims 1-6, wherein the piston (30) comprises a U-shaped structure (104).

9. A system according to one of the preceding claims, wherein the cochlearr electrode (4) is provided with a force sensor.

10. A system according to one of the preceding claims, wherein the system comprises an insertion tool (2) comprising a handle (32) and a visual insert depth indicator (34).

11. A system according to one of the preceding claims, wherein the system comprises a stabilizing unit.

12. A system according to one of the preceding claims, wherein the system comprises a detection unit configured to measure the rate of change of insertion force during operation of the tool.

**13.** A system according to one of the preceding claims, wherein the system comprises a control unit configured to make sure that insertion is continued until the real-time rate of change of force goes below or above a predefined quantity (threshold).

**14.** A system according to one of the preceding claims 9-13, wherein the force sensor is arranged in the casing (32) of the insertion tool (2) and comprises a one-axis sensor.

**15.** A system according to one of the preceding claims, wherein the system is configured to measure the internal friction of the insertion tool (2).

Fig. 1A

Fig. 1B

Fig. 1C

Fig. 2

Fig. 3A

Fig. 3B

Fig. 3C

Fig. 4

Fig. 5

Fig. 6A

Fig. 6B

Fig. 6C

Fig. 6D

EP 3 513 834 A1

Fig. 7A

Fig. 7B

Fig. 7C

Fig. 7D

Fig. 7E

Fig. 7F

Fig. 7G

Fig. 8B

Fig. 8A

Fig. 8C

Fig. 9D

Fig. 9C

Fig. 9A

Fig. 9B

Fig. 10E

Fig. 10A

F

T

Fig. 10B

F

T

Fig. 10C

F

T

Fig. 10D

2

132   134   136   138

140

142

Fig. 10E

138   144

Fig. 11A

Fig. 11B

Fig. 11C

Fig. 12A

158

160

20

156

F'

154

4

2

6

F

C

F₂

F₁

T

Fig. 12B

F

F₂

B

A

Fig. 12C

Fig. 13A

F

Sensor force

Friction force

Deduced force

Insertion depth

F

Fig. 13B

Insertion depth

S

ΔF

Δ depth (or Δ time)

Fig. 13C

Europäisches
Patentamt

European
Patent Office

Office européen
des brevets

# EUROPEAN SEARCH REPORT

Application Number

EP 18 18 5673

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2013/172901 A1 (BOZORG GRAYELI ALEXIS [FR] ET AL) 4 July 2013 (2013-07-04)<br>* abstract; figures 1-6 *<br>* paragraphs [0023] - [0049] *<br>----- | 1,4-15<br><br>2,3 | INV.<br>A61N1/05<br>A61N1/36<br>A61N1/372 |
| Y<br><br>A | US 2011/009877 A1 (THENUWARA CHULADATTA [US] ET AL) 13 January 2011 (2011-01-13)<br>* abstract; figures 1-20 *<br>* paragraphs [0038] - [0099] *<br>----- | 2,3<br><br>1,4-15 | ADD.<br>A61B17/00<br>A61B17/34 |
| A | US 2010/114288 A1 (HALLER MATTHEW I [US] ET AL) 6 May 2010 (2010-05-06)<br>* abstract; figures 1-9 *<br>* paragraphs [0031] - [0083] *<br>----- | 1-15 | |
| A | US 2011/319974 A1 (THENUWARA CHULADATTA [US] ET AL) 29 December 2011 (2011-12-29)<br>* abstract; figures 1-15 *<br>* paragraphs [0048] - [0129] *<br>----- | 1-15 | |
| | | | TECHNICAL FIELDS SEARCHED (IPC)<br><br>A61N<br>A61B |

The present search report has been drawn up for all claims

| Place of search | Date of completion of the search | Examiner |
|---|---|---|
| Munich | 26 February 2019 | Mendelevitch, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 18 18 5673

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2019

| Patent document cited in search report | | Publication date | Patent family member(s) | | Publication date |
|---|---|---|---|---|---|
| US 2013172901 | A1 | 04-07-2013 | EP | 2595568 A1 | 29-05-2013 |
| | | | US | 2013172901 A1 | 04-07-2013 |
| | | | WO | 2012010783 A1 | 26-01-2012 |
| US 2011009877 | A1 | 13-01-2011 | EP | 2451366 A1 | 16-05-2012 |
| | | | US | 2011009877 A1 | 13-01-2011 |
| | | | WO | 2011005993 A1 | 13-01-2011 |
| US 2010114288 | A1 | 06-05-2010 | NONE | | |
| US 2011319974 | A1 | 29-12-2011 | EP | 2585016 A1 | 01-05-2013 |
| | | | US | 2011319974 A1 | 29-12-2011 |
| | | | WO | 2011163443 A1 | 29-12-2011 |

EPO FORM P0459

For more details about this annex : see Official Journal of the European Patent Office, No. 12/82

**EP 3 513 834 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4207441 A
- US 4532930 A